# EUROPEAN PATENT SPECIFICATION

(11) **EP 1 664 296 B1**
(45) Date of publication and mention of the grant of the patent: **25.02.2015**
(21) Application number: 04772731.8
(22) Date of filing: 27.08.2004
(51) Int. Cl.: C12N 15/11, C12Q 1/68, G01N 33/531, G01N 37/00, G01N 33/543

(54) **METHOD FOR QUANTIFYING TARGET SUBSTANCE USING a PROBE CARRIER**
VERFAHREN ZUR QUANTIFIZIERUNG EINER ZIELSUBSTANZ UNTER VERWENDUNG eines SONDENTRÄGERS
PROCEDE DE QUANTIFICATION DE SUBSTANCES CIBLES AU MOYEN D'Un SUPPORT DE SONDE

(30) Priority: 28.08.2003 JP 2003209247
(43) Date of publication of application: 07.06.2006
(73) Proprietor: CANON KABUSHIKI KAISHA, Ohta-ku Tokyo 146-8501 (JP)
(72) Inventor: YAMAMOTO, Nobuko, c/o Canon Kabushiki Kaisha, Ohta-ku, Tokyo 146-8501 (JP)
(74) Representative: TBK
(86) International application number: PCT/JP2004/012782
(87) International publication number: WO 2005/021753

(56) References cited:
- WO-A-02/063300
- WO-A-03/025580
- WO-A-2004/099430
- WO-A1-00/42434
- JP-A- 2001 511 361
- JP-A- 2001 522 998
- US-A1- 2002 004 204
- US-A1- 2002 147 330
- US-A1- 2003 040 107

## Description

### TECHNICAL FIELD

The present invention relates to an evaluation method using a probe carrier used for the evaluation of the content of a target substance in a solution.

More particularly, it relates to a quantification method using a probe carrier for quantification of expressed genes.

### BACKGROUND ART

In recent years, methods of analyzing gene structures have been making remarkable progress. The structures of many genes, including human genes, have been elucidated. For analysis of such genes, DNA chips (DNA microarrays) have come to be used, which are constituted by spotting and immobilizing DNA fragments (hereinafter called "probe DNA") of more than thousands to ten thousands of different kinds in lines on a substrate, such as a microscope slide glass (for example, Japanese Patent Application Laid-Open No. H11-187900 and so forth).

Analyses of the amount of gene expression have come to serve for drug discovery, disease prediction, disease diagnosis, decisions of therapeutic policies and so forth. Likewise, protein chips are also used for quantification of expressed proteins.

For quantifying a DNA in a sample solution using the above-mentioned DNA microarray, the amount of fluorescence of a hybrid with labeled sample DNA that binds to this microarray is measured.

In conventional arrays, only one probe-binding site was allocated for one gene, and each site contains almost the same amount of immobilized probes.

However, the DNA content in a sample varies greatly with genes; in many cases, the high content and the low content differ 100 to 1000 times.

For this reason, to evaluate the amount of DNA of interest in a sample, it is necessary to perform analysis by preparing a highly-sensitive detection system with a wide dynamic range and also by preparing a carrier on which probes were immobilized in an amount sufficient to the amount of DNA in a sample, or by diluting the sample to a suitable concentration in relationship with the amount of the probes.

Further, for carrying out quantification at a plurality of probe-binding sites simultaneously using a solution containing DNAs of which concentration are different about 1000 times, simultaneous measurement is difficult with conventional microarrays of which spots contain probes of almost the same amount. This is because some probes become short in quantity for target substances present in a large amount, while some are present in an extremely excessive amount for target substances present in a small amount.

Moreover, when such an array was used, it is impossible to quantify the amount of fluorescence from each spot in the same dynamic range; the intensity of fluorescence had to be measured for every site individually by changing the dynamic range.

### DISCLOSURE OF THE INVENTION

The present invention provides a method using a probe carrier having a novel configuration that allows simple and convenient measurement of the content of a target substance and a method of quantifying a target substance using the carrier. The carrier further enables easy evaluation of the content of a target substance even with an inexpensive line sensor, area sensor and the like.

That is, the present invention is defined in claim 1.

The present invention provides a configuration capable of quantitatively evaluating the content of a target substance by adding up the amounts of signals from the probe spots of the same kind present in one divided area using a simple and convenient sensor such as a line sensor or an area sensor.

Further, the aforementioned probe carrier is configured such that two or more kinds of target substances can be evaluated.

The amount of probes in different plural areas varies according to the target substances to be detected.

The plural areas may be aligned in a first direction and adjacent areas may be separated in a direction vertical to the first direction.

The number of the immobilized probe molecules per spot is practically equal among all kinds of probes.

Preferably, the probe is a nucleic acid. The number of the immobilized probe molecules may be of the same order to the lowest number of mRNA molecules of target genes present in a sample.

The number of spots in each area may be proportional to an average expression amount of the target gene having a sequence complimentary to the probe.

The amount of probes immobilized may vary between different areas.

Application of probes to the carrier for immobilization may be performed by an ink jet method.

Preferably, the number of spots in each of the areas differs 100 to 1000 times between the maximum and minimum.

The carrier may be tape-shaped.

The carrier may be a plate-shaped substrate.

Further, a method of the present invention for evaluating the content of target substances in a solution using a probe carrier on which probe molecules capable of specifically binding to the target substance are immobilized in predetermined locations on the carrier includes the steps of
preparing a probe carrier having two or more separated areas on the carrier, wherein, in each area, probe molecules of the same kind are immobilized as two or more spots and probes of different kinds are not immobilized ;
contacting the carrier with the sample solution to bind the target substance to the probe molecules;

The content of the target substance in the solution is evaluated by adding up the signal intensity in the area.

The integration of the signal intensity may be performed with a line sensor or an area sensor. The number of probe molecules immobilized on the probe carrier varies according to the kind of the probe. Two or more kinds of target substances in the solution are evaluated.

The amount of probes immobilized on the probe carrier **is** fixed respectively at 1.0 to 2.0 times as much as the expected amounts of the target substances in the solution.

The probe carrier may be a tape-shaped carrier, and the step of contacting the probe and the sample solution includes the step of contacting a part of the probe carrier with the solution and the step of sequentially changing the portion in contact with the solution by moving the carrier in relation to the solution.

Thus, according to the present invention, a method can be provided whereby quantitative determination of a target substance can be evaluated in a simple and convenient manner by detecting the signal intensity related to the amount of the target substance bound to a plurality of spots disposed in one area.

Further, by using a probe carrier having probes capable of binding to a target substance where the probes are disposed in one of divided areas in an amount corresponding to the amount of a target substance of interest, respective hybrids can be controlled to the amount suitable for measurement of the signal intensity, i.e. measurement of fluorescence, thereby enabling simple and convenient measurement without the need of changing dynamic ranges.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

The accompanying drawings, which are incorporated in and constitute a part of the specification, illustrate embodiments of the invention and, together with the description, serve to explain the principles of the invention.
Fig. 1 illustrates one embodiment of the probe carrier in accordance with the method of the present invention where the number of spots varies with the kinds of probes immobilized;
Fig. 2 illustrates another embodiment of the probe carrier in accordance with the present invention where the carrier is divided to a plurality of areas, where the same kind of probe molecules are immobilized as a plurality of spots in respective areas and the number of spots varies with the kinds of the probes;
Fig. 3 illustrates a probe carrier
   wherein the carrier is divided into a plurality of areas, and the same kind of probe molecules are immobilized in each area and each area contains the same number of spots but the number of probe molecules per spot varies with the kinds of the probes;
Fig. 4 illustrates another embodiment of the probe carrier in accordance with the present invention;
Fig. 5 illustrates another embodiment of the probe carrier in accordance with the present invention;
Fig. 6 illustrates another embodiment of the probe carrier in accordance with the present invention;
Fig. 7 illustrates another embodiment of the probe carrier in accordance with the method of the present invention;
Fig. 8 illustrates another embodiment of the probe carrier in accordance with the method of the present invention;
Fig. 9 illustrates another embodiment of the probe carrier in accordance with the method of the present invention and the use of the probe carrier;
Fig. 10 schematically illustrates the manufacturing method of the probe carrier in accordance with the method of the present invention;
Fig. 11 schematically illustrates the configuration of a cassette having the probe carrier in accordance with the method of the present invention; and
Fig. 12 schematically illustrates a tape-like probe carrier in accordance with the method of the present invention in the manufacturing process stage.

### BEST MODE FOR CARRYING OUT THE INVENTION

Preferred embodiments of the present invention will now be described in detail in accordance with the accompanying drawings.

Hereafter, a method of evaluating the content of a target substance of the present invention using the probe carrier will be explained in detail.

The method of evaluating the content of a target substance in accordance with the present invention is done according to claim 1 and in the following steps:
(1) The step of preparing a probe carrier having two or more separated areas provided on the carrier, wherein, in each area, probes of the same kind are immobilized as one or more spots and probes of different kinds are not immobilized and in at least one area probes of the same kind are immobilized as two or more spots;
(2) The step of contacting the carrier with the solution to bind the target substance to the probes; and
(3) The step of measuring the intensity of the signal related to the target substance bound to the .probes.

Hereafter, each step will be explained in detail.

### <Preparation of Probe Carrier>

First, the step of preparing a probe carrier having probes capable of specifically binding to a target substance bound to known locations on the carrier will be explained. Exemplary of this step is, for example, fabrication of a DNA microarray and the like. It is typical configurations of DNA array that various DNA probes are adsorbed on the surface of a glass substrate treated suitably for immobilizing DNA by a strong bond, such as covalent bond. Also there are resin substrates and substrates coated with thin metal film.

In the present invention, a windable tape-shaped base material may be used. In that case, a method may be adapted of contacting the sample solution with only part of this tape-shaped base material and moving sequentially the position for contacting with the solution, which will be described later.

### <Target substance and Target-specific probe>

In the present invention, a probe to be immobilized onto a carrier is capable of specifically binding to a specific target substance. Further, examples of this probe include oligonucleotides and polynucleotides, or other polymers, capable of recognizing a specific target. The term "probe" as used herein refers both to a molecule having a probe function, such as a polynucleotide molecule, and a population of molecules having the same probe function, such as polynucleotides of the same sequence, including a molecule called a ligand. Moreover, a probe and a target are often used interchangeably, and can be bound to each other as part of ligand/anti-ligand (which may also be referred to as a receptor) pair, or may come to bind. For the purpose of the present invention, a probe and a target may include naturally occurring bases or their analogs.

One example of a probe supported on a carrier is an oligonucleotide containing a nucleotide sequence hybridizable with a target nucleic acid, which has a binding portion to be linked to the carrier surface via a linker. The position of such a binding portion in the probe oligonucleotide molecule is not limited as long as it does not hinder the desired hybridization reaction.

A probe usable for the probe carrier manufactured by the method of the present invention is appropriately selected according to the purpose of use. Preferable probes for the purpose of advantageously embodying the present method are DNA, RNA, cDNA (complementary DNA), PNA, oligonucleotides, polynucleotides, other nucleic acids, oligopeptides, polypeptides, proteins, enzymes, enzyme substrates, antibodies, epitopes of antibodies, antigens, hormones, hormones, receptors, ligands, ligand receptors, oligosaccharides and polysaccharides. A combination of two or more thereof may be used as necessary.

As used herein, "probe carrier" refers to a carrier having a plurality kinds of probes immobilized onto the carrier surface (including the surface of the inner walls of hollow members or tubular carrier members) as independent areas, such as dot-like spots; and "probe array" refers to a carrier having such probes aligned at a predetermined interval.

It is desirable that a probe has a structure capable of binding to the surface of a carrier and that immobilization of a probe onto a carrier be performed via this bindable structure. In that case, preferably, the structure of the probe enabling binding to a carrier surface is formed by the treatment that introduces an organic functional group to the probe, such as amino group, mercapto group, carboxyl group, hydroxyl group, acid halides (haloformyl group; -COX), halides (-X), aziridine, maleimide group, succinimide group, isothiocyanate group, sulfonyl chloride group (-SO₂Cl), aldehyde group (formyl group; -CHO), hydrazine, and acetamide iodide. Further, the surface of the carrier may be subjected to necessary treatment according to the binding structure of the probe.

### <Disposition and configuration of probes on a carrier>

The probe carrier usable in the method in accordance with the present invention is characterized by having a plurality of independent areas, wherein in each area probes of the same kind are immobilized as two or more spots and probes of different kinds are not immobilized therein.

In order to immobilize more kinds of probes as separated spots, the area of each spot is preferably 10 µm² to 500 µm².

In addition, as for each probe, the following configurations variably control the amount of probes to be bound to a carrier:
(B) The number of spots per unit area in each area is fixed and the sizes of the areas are different.
(C) The size of each area and the number of spots per unit area of each area are different from area to area.

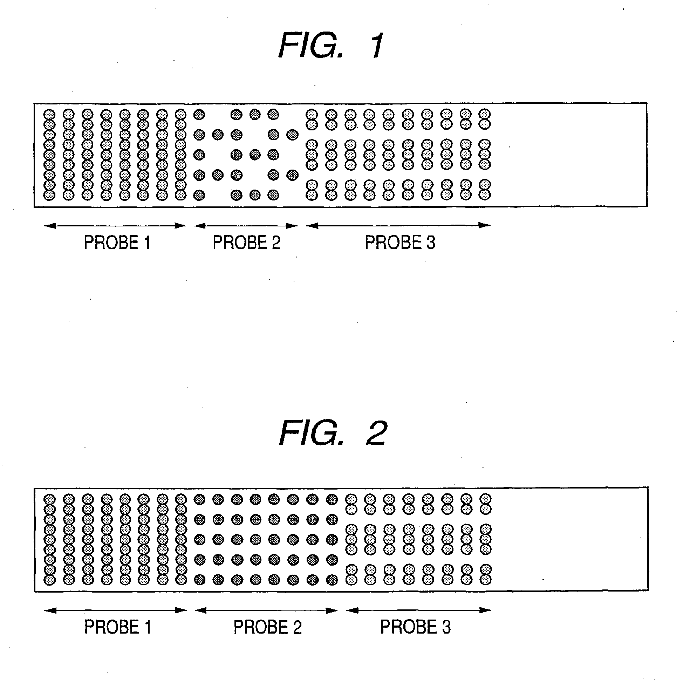

One embodiment is, as shown in Fig. 1, a configuration in which the same probes are immobilized in the width direction and different probe species are disposed in the longitudinal direction of a flat substrate.

In addition, the areas where the same probes are immobilized may be different in size (area). In addition, the densities of the spots may vary with locations.

Another embodiment of the probe carrier in accordance with the method of the present invention is, as shown in Fig. 2, to control the spot density of respective areas such that the spotted areas become the same.

In this case, the concentration of each probe solution to be spotted is the same regardless of the probe species.

### <Method of forming spots>

For the method of forming spots of probes immobilized on a carrier, any conventionally known methods may be used.

For example, spots can be formed using an ink jet method or a photolithography technique.

Formation of a spot by an ink jet method is performed by ejecting a solution containing a probe as a minute droplet to apply the droplet onto a probe carrier. This method makes it possible not only to have the landing diameters (which practically correspond to the spot diameters) uniform but also to easily change the amount of probes present in a spot area by changing the concentration of the solution to be ejected, whereby the probe carrier can be suitably fabricated.

Further, using an ink jet method, it is also possible to easily form spots having different landing diameters (spot size) with an ejection solution of a certain probe concentration by appropriately changing the ejection energy etc. In this case, the probe density in a spot (the number of probe molecules/spot area) is the same for each spot; it is possible to make the average luminance of 80% of probe hybridization almost same for example. This provides a quantification method without the need of changing the dynamic range.

### <Adjustment of the immobilization amount of probe and amount of target substance>

In the present invention, it is effective to predict the order of the immobilization amount of the probe on a probe carrier in advance from the sample solution containing a target substance. In some cases, the concentration of the solution to be contacted is adjusted in the light of the amount of the probe on a probe carrier. However, this cannot adjust respective concentrations of respective target substances. Thus, for quantitative analysis of respective target substances, it is very important to adjust the immobilization amount of the probe on a probe carrier with respect to the amount of a target substance.

Of course, strict adjustment of each component amount is not necessarily required. For example, for analyzing expression of two different genes A and B, when it is expected that the expected expression amount of the gene A is nearly 100 times as much as that of the gene B, the probe carrier may have probes different in the immobilization amount by about 100 fold. To be more specific, when 0.01 µmole mRNA and 1 µmole mRNA expressed from gene A and gene B respectively are present in a solution, a probe carrier may be prepared which has 0.012 µmole of nucleic acid probe A', capable of specifically binding to the mRNA expressed from gene A, and 1.2 µmole of nucleic acid probe B', capable of specifically binding to the mRNA expressed from gene B, immobilized on the carrier.

### <Step of contacting a probe carrier with a solution containing a target substance>

Next, the step of contacting a probe carrier with a solution containing a target substance will be explained.

In this step, a probe carrier is contacted with a solution containing a target substance. As previously described, the amount of the target substance in the solution is predicted in advance, and then the solution is contacted with the probe carrier, so that the target substance binds to a probe on the probe carrier. When both the target substance and the probe are nucleic acid, nucleic acid hybrids are formed by a hybridization reaction. The conditions under which the hybridization reaction occurs vary with the nucleic acid to be used, but similar conditions may be used to those for hybridization on an ordinary solid substrate.

In addition, when a windable tape-shaped base material (as will be described hereafter) is used, a sample solution is contacted with only part of this tape-shaped base material. After a hybridization reaction of the probe present in the portion in contact with the solution and the target substance in the solution occurs sufficiently, the tape-shaped probe carrier will be moved in relation to the solution such that another part of the probe carrier is contacted with the solution. By repeating this action, the binding between the probes and the target substances is observed. The detailed procedure will be explained later.

### <Measuring Step of Signals from bound target substance>

This step includes a method measuring the amount of a target substance by binding of a fluorescent-labeled, target substance to a probe on a probe carrier by fluorometry.

For example, the amount of a target substance is calculated by counting the number of spots emitting signal and adding up the signal intensity of each spot. Instead of measuring the signal intensity per spot, it is also possible to read signal in every row of spots using so-called line senser. In this case, it is important to arrange one kind of probe in the same row; preferably, the probe for expression in a small quantity and thus should be provided as one spot is present as only one spot in one row.

The intensity of the signal per each area corresponds to the amount of expression of respective genes. It is preferable to use an area sensor for measurement in terms of miniaturization and low cost.

In some cases, fluorescent measurement may lack reproducibility because the fluorescence of the labeling agent (pigment etc.) may fade by one measurement. The conventional measurement changing the measuring range results in repeated measurement of the same site; causing a problem in reproducibility and in intensity comparison between respective ranges. In the method of the present embodiment, one measurement in the same dynamic range is possible. In addition, a simple and convenient measuring apparatus that does not require range adjustment function and the like can be used.

However, in any case, it is preferable to select optimal combination, based on examination of the relationship between the amount of expression and the sensitivity of the sensor.

Hereafter, the embodiments of the present invention will be explained.

### Embodiment 1

The following describes an embodiment in which the carrier is a flat substrate like a slide glass.

In this embodiment, the amount of the immobilized probe on the carrier is larger than that of the corresponding target substance of interest in a sample solution and the amounts of the immobilized probes are different according to corresponding target substances.

### <Test plate for quantification of biological sample and its use>

The test plate according to the present embodiment comprises a glass substrate of a commercial size on which two or more areas are formed where different probes are disposed respectively.

The test plate can be manufactured by attaching a liquid containing a probe to the carrier using application means while moving the means or carrier. For such liquid application means, a liquid ejection apparatus that ejects a liquid from the openings of various pipettes or nozzles can be used.

In this embodiment, it is preferable, as shown in Fig. 1, the same probes are immobilized in the width direction and different probe species are disposed in the longitudinal direction of the substrate. In addition, the areas where the same probes are immobilized may be different in size (area). In addition, the densities of the spots may vary with locations.

Further, these density variation may be made by the changes in the number of spots applied by ink jet etc.

When a mode is employed in which binding reaction between the probe and the substrate occurs on the substrate, the liquid containing the probe is applied to the substrate to form an attachment area. In this state, the probe starts to react with the carrier, whereby a probe immobilization area is formed there. While when a specific treatment for immobilizing the probe to the substrate is required, the substrate on which the probe liquid was attached is subjected to appropriate immobilization treatment.

### <Carrier for gene quantification and its use>

An embodiment of the probe-carrier for gene quantification is that shown in Fig. 1, where solutions of various probes are prepared to the same concentration and the spot number is altered to reflect the expression amount of respective target genes in cells, e.g., human tissue, that is, large spot number for a highly expressed gene and small spot number for a gene with reduced expression.

In this case, if the number of the molecules of a gene with the lowest expression among the expressed genes in a sample and the number of the probe molecules in one spot are made to agree and the agreed value is used as the unit, quantification of genes that are greatly different in expression becomes possible by setting the number of spots to 100, 1,000 etc., according to the expression degree of the genes.

Further, the total amount of one probe immobilized on the aforementioned probe carrier is 1.0 to 2.0 times as much as the amount expected for its target substance in the solution.

Several specific examples of a probe carrier having the above-described configuration will be illustrated below.

### Example 1

### Preparation of a plate for quantification of gene expression

A commercially available slide glass was set on an ink jet printer in the same manner as paper. Six kinds of oligonucleotide probe DNAs having different sequences were filled into six ink cartridges of this ink jet printer and printing was carried out on a slide glass as shown in Fig. 1. The printed slide glass was heated to 180°C to immobilize the probe DNA on it.

### <Analysis of expression amount using a plate for quantification of gene expression>

Reaction between a target substance contained in a sample and a probe immobilized on a carrier can be signalized by various methods. Usually a labeling substance is used, i.e. a fluorescent substance that can emit optical signal detectable by a sensor and the like.

Amplification and fluorescent-labeling of mRNA can be carried out by a conventional in vitro transcription method (e.g., the MegaScript kit, Ambion Ltd.).

When such a sample solution is contacted with the probe carrier and then the reaction between the fluorescent labeled target substance and the probe is detected as fluorescence, the amount of reaction between the probe and the target substance in each probe immobilization area can be determined by moving the probe carrier or the detection means such as a sensor.

When using a liquid ejection apparatus to apply a liquid to a substrate such as a probe solution, it is advantageous to use a liquid ejection apparatus that has a plurality of liquid-ejection members each comprising a liquid container, a nozzle for ejecting the liquid connected to the liquid container and means of generating liquid ejection energy for ejecting liquid from the nozzle, of which number may be selected depending on the kinds of liquids to be ejected.

Means of generating liquid ejection energy include various methods, such as a piezoelectric method and a heating method. However, in arranging a large number of liquid ejection parts in high density, each of which needs to be provided independently, a heater element capable of generating thermal energy, causing film boiling by heating a liquid and ejecting a liquid from the opening of the nozzle using the pressure may be suitably used.

More preferable is a heater element having a structure in which a bubble produced by film boiling communicates with the open air via the opening of the nozzle.

### Embodiment 2

The following embodiment illustrates the one in which the carrier is composed of a windable tape-shaped material.

In this embodiment, probes on a carrier are present in an excessive amount to the target substance of interest in a solution. In this embodiment, instead of contacting the whole probe-bearing carrier simultaneously, only part of the carrier is contacted with the solution sequentially, whereby the amount of the target substance in the solution is estimated.

### <Test tape for quantification of biological samples and its use>

The test tape for quantification of biological samples in accordance with the present embodiment includes two or more areas on which different probes are disposed in the longitudinal direction of the long substrate.

Meanwhile, the test tape of the present embodiment may also be provided in the form of pieces, such as a thread, a string, a belt and a chip. Further, the tape may be wound around a core to be provided as a roll or a bobbin and used continuously in the unwound state, or used as a piece that has been cut in an appropriate length.

Moreover, the test tape for quantification of biological samples in accordance with the present embodiment can be manufactured by attaching a liquid to a carrier while moving the probe solution-applying means along the longitudinal direction of the tape. For such means for applying a liquid, a liquid ejection apparatus that ejects a liquid from the openings of various pipettes or nozzles can be used.

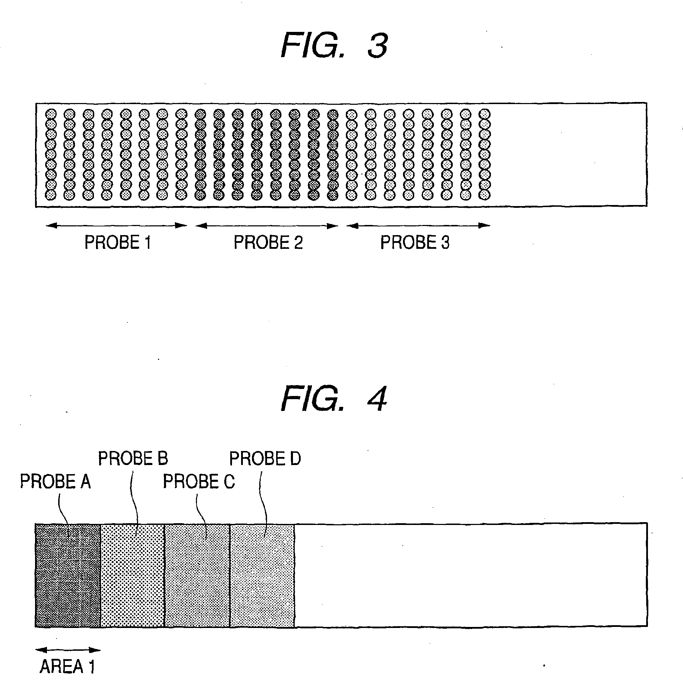

As shown in Fig. 4, the test tape for quantification of biological samples has the same probes immobilized in its width direction and plural different probe species immobilized in its longitudinal direction. The areas in which different probes are immobilized respectively may have the same area (size) and the spot density may vary depending on the areas.

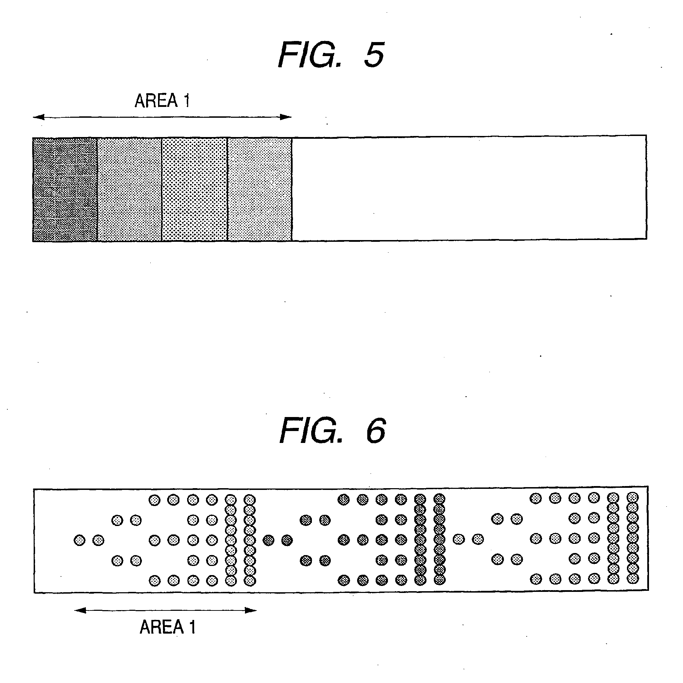

Moreover, as shown in Fig. 5, in one area where the same substance is immobilized, the density of the immobilized substance may vary in the longitudinal direction.

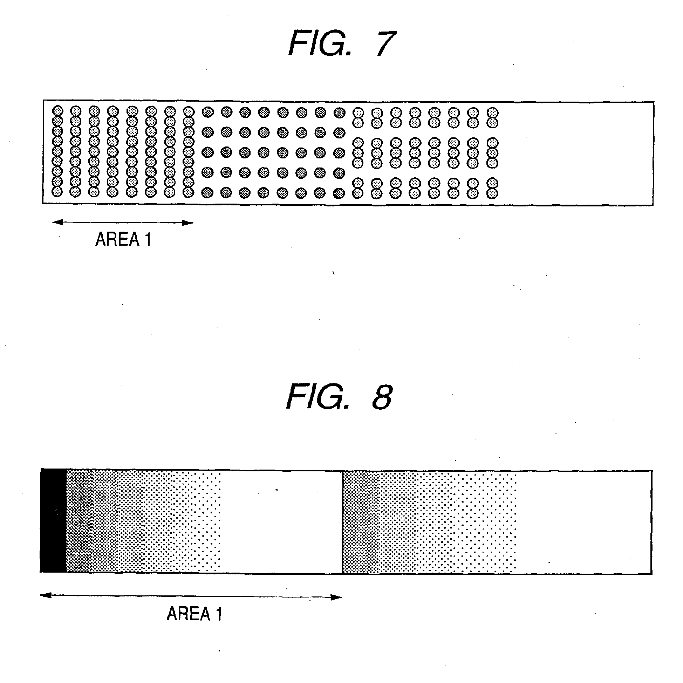

What is more, such a density change may be achieved by changing the number of spots, by using an ink jet etc. (Figs. 6 and 7).

Such density change can also be automatically made using a gradation function of ink jet printing.

Fig. 8 shows an example of a probe carrier on which probe density continuously varies in the longitudinal direction of the carrier.

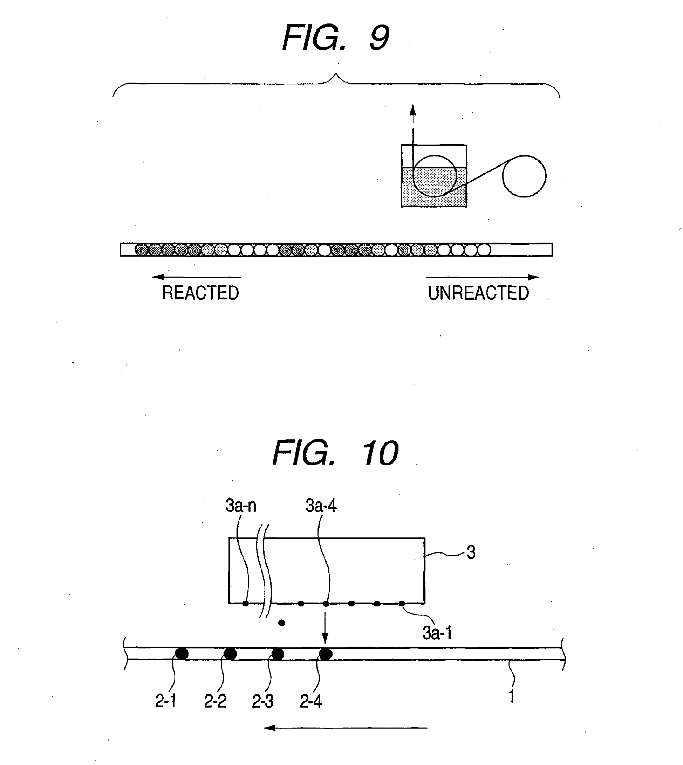

In addition, a tape-shaped carrier includes a string-shaped one as shown Fig. 9.

Fig. 10 shows an example of the step of applying a liquid containing a probe while moving a liquid ejection apparatus relatively to a string-shaped substrate. A liquid ejection apparatus 3 has nozzle openings 3a-1 to 3a-n, each of which ejects a liquid containing a different probe, and these nozzles are disposed linearly in the longitudinal direction of the carrier. The nozzle alignment of the liquid ejection apparatus 3 may be as follows: A plurality of nozzles are disposed in the direction intersecting at right angles to the longitudinal direction of the carrier, and the position of nozzles in that direction can be changed as necessary so that nozzles facing the carrier can be exchanged. This makes it possible to apply many kinds of probe liquids from as many nozzles as possible. In the example shown in Fig. 10, a carrier 1 moves relatively in the direction of the arrow to the liquid ejection apparatus 3. First, a first probe solution is applied to the carrier from the nozzle 3a-1 to form attachment area 2-1. Next, different probe solutions are ejected one by one from nozzles 3a-2 to 3a-4 to form liquid attachment areas 2-2 to 2-4. Fig. 10 shows the state when a liquid has been ejected from the nozzle opening 3a-4. Further ejection is performed as necessary to 3a-n to form attachment areas for different probes to 2-n. Further repetition of such operations enables continuous formation of a large number of probe carriers of the same configuration.

Meanwhile, when a carrier is provided as a piece of a certain rigidity, application of a liquid to it using a liquid ejection apparatus is enabled by conveying the piece by a suitable support member such as a belt conveyer.

When a mode is employed in which binding reaction between the probe and the substrate occurs on the substrate, the liquid containing the probe is applied to the substrate to form an attachment area. In this state, the probe starts to react with the carrier, whereby a probe immobilization area is formed there. While when a specific treatment for immobilizing the probe to the substrate is required, the substrate on which the probe liquid was attached is subjected to appropriate immobilization treatment.

A carrier of thread, string, or belt form, the carrier may be fed to the liquid ejection apparatus as a bobbin or roll wound around a core. Such a carrier may be suitably fed to the liquid ejection apparatus by continuously unwinding by moving means in combination of a roller, guide and the like. An example of such a form is shown in Fig. 10. A thread-shaped carrier 1, provided as a roll, moves along a moving path with a roller pair, a guide roller and the like to the position where the liquid ejecting apparatus 3 is placed where a liquid containing a probe is applied to the carrier. Unwinding from the roll can be done by rotating the roller pair with suitable driving means. In that case, applying a suitable backward tension to the roll can prevent the substrate from slacking. Preferably, the roll may include a dummy portion at the leading end thereof such that the dummy portion of the predetermined length passes through the installation section of the liquid ejection apparatus before the carrier part is fed into the apparatus.

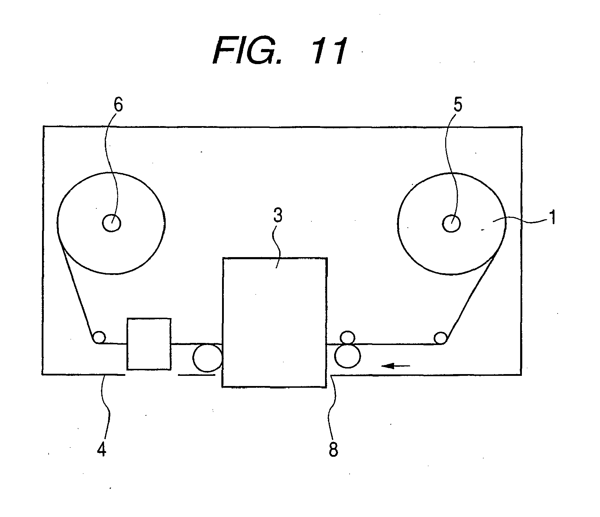

A cassette housing carrier rolls therein is a suitable mode for applying probe to a carrier unwound from a roll using a liquid ejection apparatus and recovering the resulting probe carrier as a roll. Fig. 11 schematically shows a cassette having such a configuration placed in a manufacturing apparatus provided with a liquid ejection apparatus. This cassette has a cabinet 4 incorporating a member 5 and a take-up reel 6 for housing a roll-shaped carrier together with various kinds of guide rollers. A roll-shaped carrier 1 moves by being wound around the reel 6 by rotational drive of the driving roller on the side of the manufacturing apparatus and of the reel 6. A probe solution is applied to this carrier at the opening 8 of the cabinet 4 from the liquid ejection apparatus 3. The part to which a probe solution was applied may be subjected to a drying process or a probe-immobilizing process before wounded to the reel 6, as required. Such processes may be applied via another opening provided in the cabinet 4 from outside the cassette.

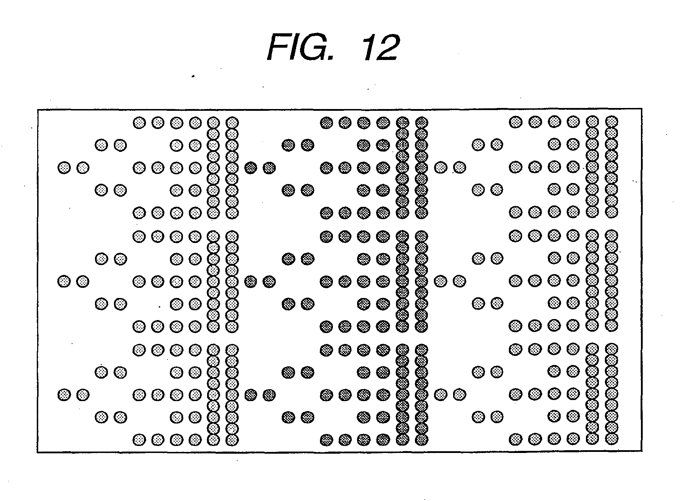

A probe carrier wound to the reel 6 may be used in various forms. For example, the probe carrier may be taken out as a roll from the cassette to be shipped as a product; or may be unwound from the roll and cut at the predetermined length. As shown in Fig. 12, a plurality of spot arrangements of Fig. 6 may be made in parallel. This may be processed into the Fig. 6 spot arrangement by cutting the roll that has been wound onto the reel 6. Further, the cassette may be used as a supply opening in analytical procedures.

It is necessary that the probe information (genes or proteins such as antibody) corresponds with the address on the tape-shaped carrier configured in a cassette. For address, by forming a magnetic recording layer on one surface of the tape, it is possible to store magnetic data information in this magnetic recording layer. Alternatively, information on the substance immobilized by knowing the elapsed time when the tape is scanned at a certain speed.

Shown below is an example of an long probe carrier having the above-described configuration.

### Example 2

### Fabrication of a plate for quantification of gene expression

A roll of cellulose-nitrate paper was set in an ink jet printer in the same manner as paper. Six kinds of oligonucleotide probe (DNA) having different sequences were filled into six ink cartridges of this ink jet printer and printed on the cellulose-nitrate paper as shown in Fig. 1.

Specifically, 1 µmole solutions of different probes were filled separately in the liquid container of the liquid ejection apparatus and the cellulose-nitrate paper was moved along the alignment line of nozzle openings of the liquid ejection apparatus, whereby respective solutions were attached to the predetermined part. A probe carrier was thus obtained having areas where different oligonucleotides are immobilized in the longitudinal direction.

Then the nitrocellulose paper was wound again after probe printing as shown in Fig. 11, and the probe DNA was immobilized by heating the paper to 180°C. Subsequently, the paper roll was cut to fabricate a belt-like sheet having a pattern as shown in Fig. 6.

### <Analysis of expression amount using a tape for quantification of gene expression>

Reaction between a target substance contained in a sample and a probe immobilized on a carrier can be signalized by various methods. Usually a labeling substance is used, such as a fluorescent substance that can emit optical signal detectable by a sensor and the like.

mRNA amplification and fluorescent-labeling methods are carried out by the conventional in vitro transcript method (e.g., the MegaScript kit, Ambion Ltd.), as in Example 1.

When such a sample solution is contacted with the probe carrier and then the reaction between the fluorescent labeled target substance and the probe is detected as fluorescence, the presence or absence of reaction between the probe and the target substance in each probe immobilization area can be determined by moving the long probe carrier or the detection means such as a sensor.

Further, the roll housing part of the cassette shown in Fig. 11 can be set with a sample roll for measurement, i.e., a probe carrier contacted with a sample solution and in a state suitable for optical detection of the presence or absence of reaction between the probe and the target substance. Carrying out a winding operating with a winding reel 6 at the timing required for measurement, it is possible to optically measure the presence or absence of a reaction by sensing means, such as a sensor, at the opening 8. At this time, in the case of the array having an arrangement as shown in Fig. 5, simple and convenient measurement of signal intensity is also possible by measuring the distance between the signal generating point and the point where signal disappears completely and calculating from the distance information the signal intensity as an area.

The detection apparatus that is used with a cassette may include a fitting part for a cassette having within a cabinet the aforementioned housing part for housing a sample roll wound to a feed reel, a take-up reel for taking up the sample roll fed from the feed reel and an opening provided along the moving path of the measurement sample extending between the roll housing part and the take-up reel; driving means for driving the take-up reel of the cassette arranged in the fitting part; and aligning means for aligning the signal detection means of the detection apparatus to a position where it can detect the signal from a probe immobilization area of the measurement sample that is driven to move on the moving path by the driving means via the opening.

Although the above-described examples illustrate embodiments when a line sensor is used, the present invention is not limited to this configuration. When local sensors, such as an area sensor, are used, different probes can be disposed in the end side direction.

## Claims

1. An in-vitro method of evaluating the contents of two or more fluorescent-labeled target substances in a solution, comprising the steps of:
preparing a probe carrier on which probes each capable of specifically binding to one of the target substances are immobilized in predetermined locations in a plurality of separate areas on the carrier;
contacting the carrier with the solution to bind the target substances to the probes; and
measuring the intensity of signal related to the target substances bound to the probes by fluorometry;
wherein the probe carrier has a plurality of separate areas, wherein in each area the same probes are immobilized as two or more spots and different probes are not immobilized therein, wherein the amount of target substances is calculated by counting the number of spots emitting signal and adding up the signal intensity,
wherein, as for each probe, one of the following configurations (B) or (C) is applied to vary the amount of probes bound to the carrier:
(B) the number of spots per unit area in each area is fixed and the sizes of the areas are different, or
(C) the size of each area and the number of spots per unit area of each area are different from area to area,
wherein the amount of immobilized probe molecules per spot is equal for all the spots, and
wherein the amount of probes of each kind immobilized on the carrier is 1.0 to 2.0 times as much as the expected amount of the target substances in the solution corresponding to the probes.

## Patentansprüche

1. In-vitro-Verfahren zum Evaluieren des Gehalts zweier oder mehrerer Fluoreszenz-markierter Zielsubstanzen in einer Lösung, umfassend die Schritte:
Vorbereiten eines Sondenträgers, auf welchem Sonden, die jeweils in der Lage sind, spezifisch an eine der Zielsubstanzen zu binden, in vorbestimmten Örtlichkeiten in einer Mehrzahl getrennter Bereiche auf den Träger immobilisiert sind;
Kontaktieren des Trägers mit der Lösung, um die Zielsubstanzen an die Sonden zu binden; und
Messen der Intensität eines Signals, das mit den Zielsubstanzen, die an die Sonden gebunden sind, in Beziehung steht, durch Fluorometrie;
wobei der Sondenträger eine Mehrzahl getrennter Bereiche aufweist, wobei in jedem Bereich die gleichen Sonden als zwei oder mehr Punkte immobilisiert sind und unterschiedliche Sonden nicht darin immobilisiert sind, wobei die Menge der Zielsubstanzen durch Abzählen der Anzahl an Punkten, die ein Signal imitieren, und Aufsummieren der Signalintensität berechnet wird,
wobei bezüglich jeder Sonde eine der folgenden Konfigurationen (B) oder (C) angewandt wird, um die Menge an Sonden, die an den Träger gebunden sind, zu variieren:
(B) die Anzahl der Punkte pro Einheitsfläche in jedem Bereich ist fixiert und die Größen der Bereiche sind unterschiedlich, oder
(C) die Größe jedes Bereichs und die Anzahl der Punkte pro Einheitsfläche jedes Bereichs sind von Bereich zu Bereich unterschiedlich,
wobei die Menge an immobilisierten Sondenmolekülen pro Punkt für alle Punkte gleich ist, und
wobei die Menge an Sonden jeder Art, die auf dem Träger immobilisiert sind, 1,0 bis 2,0 Mal so viel sind wie die erwartete Menge der Zielsubstanzen in der Lösung, die den Sonden entsprechen.

## Revendications

1. Procédé in-vitro pour évaluer le contenu de deux ou plusieurs substances cibles marquées par fluorescence dans une solution, comprenant les étapes consistant à :
préparer un support de sonde sur lequel des sondes, pouvant chacune se lier spécifiquement à l'une des substances cibles, sont immobilisées dans des emplacements prédéterminés dans une pluralité de zones séparées sur le support ;
mettre en contact le support avec la solution pour lier les substances cibles aux sondes ; et
mesurer l'intensité du signal relatif aux substances cibles liées aux sondes par fluorométrie ;
dans lequel le support de sonde a une pluralité de zones séparées, dans lequel dans chaque zone, les mêmes sondes sont immobilisées sous forme de deux ou plusieurs points et différentes sondes ne sont pas immobilisées à l'intérieur de cette dernière, dans lequel la quantité de substances cibles est calculée en comptant le nombre de points émettant le signal et en additionnant l'intensité de signal,
dans lequel, en ce qui concerne chaque sonde, l'une des configurations suivantes (B) ou (C) est appliquée afin de modifier la quantité de sondes reliées au support ;
(B) le nombre de points par unité de zone dans chaque zone est fixe et les tailles des zones sont différentes, ou bien
(C) la taille de chaque zone et le nombre de points par unité de zone de chaque zone sont différents d'une zone à l'autre,
dans lequel la quantité de molécules de sonde immobilisée par point est identique pour tous les points, et
dans lequel la quantité de sondes de chaque type immobilisée sur le support est de 1,0 à 2,0 fois la quantité attendue des substances cibles dans la solution correspondant aux sondes.
